# EUROPÄISCHE PATENTSCHRIFT

(11) **EP 2 077 874 B1**
(45) Veröffentlichungstag und Bekanntmachung des Hinweises auf die Patenterteilung: **14.09.2011**
(21) Anmeldenummer: 07818445.4
(22) Anmeldetag: 26.09.2007
(51) Int. Cl.: A61M 5/142, A61M 5/158, F04B 23/02, F04B 43/12

(54) **PERISTALTISCHE MIKROPUMPE MIT WECHSELBAREM PUMPENKOPF**
PERISTALTIC MICROPUMP HAVING AN EXCHANGEABLE PUMP HEAD
MICRO-POMPE PÉRISTALTIQUE COMPRENANT UNE TÊTE DE POMPE REMPLAÇABLE

(30) Priorität: 07.10.2006 DE 102006047613
(43) Veröffentlichungstag der Anmeldung: 15.07.2009
(73) Patentinhaber: Sanofi-Aventis Deutschland GmbH, 65929 Frankfurt am Main (DE)
(72) Erfinder: BASSO, Nils, 65926 Frankfurt am Main (DE); POMMEREAU, Christian, 65926 Frankfurt am Main (DE); CLARKE, Alastair, 65926 Frankfurt am Main (DE); RICHTER, René, 01062 Dresden (DE)
(86) Internationale Anmeldenummer: PCT/EP2007/008361
(87) Internationale Veröffentlichungsnummer: WO 2008/040477

(56) Entgegenhaltungen:
- EP-A- 1 045 146
- GB-A- 2 012 373
- US-A1- 2002 169 439
- US-A1- 2004 191 086

## Beschreibung

Die Erfindung betrifft eine Vorrichtung zur Injektion eines Arzneimittels in den menschlichen oder tierischen Körpern mittels eines einfach und schnell auswechselbaren Pumpenkopfes mit Motorantrieb.

Viele Arzneimittel müssen in den Körper injiziert werden. Dies gilt vor allem für solche, die bei oraler Gabe inaktiviert werden oder an Wirksamkeit entscheidend verlieren. Zu diesen Arzneimitteln zählen insbesondere Proteine (wie z. B. Insulin, Wachstumshormone, Interferone), Kohlehydrate (z. B. Heparin), Antikörper oder die meisten Impfstoffe. Zur Injektion in den Körper werden überwiegend Spritzen, Medikamentenstifte (Pens) oder Medikamentenpumpen verwendet.

Das klassische Injektionsgerät für Insulin ist die Insulinspritze. Diese findet seit Beginn der Insulintherapie Anwendung, ist aber in den letzten Jahren vor allem in Deutschland durch Einführung der Insulinpens schrittweise verdrängt worden. Dennoch sind heute die Spritzen, z. B. bei Verlust oder Defekt eines Insulinpens, unersetzlich und werden von vielen Diabetikern in Kombination mit Insulinpens verwendet. Besonders bei Reisen ist die Wartungsfreiheit und die weltweite Verfügbarkeit vorteilhaft.

Insulinspritzen unterscheiden sich in ihrer Bezeichnung und Skalierung nach der Konzentration des zu verwendenden Insulin U40 bzw. U100. Das Insulin kann sowohl aus Fläschchen als auch aus den vorgefüllten Ampullen für Insulinpens entnommen werden. Dies ermöglicht das Mischen von verschiedenen Insulinsorten und reduziert die Anzahl der notwendigen Injektionen. Bei dem Aufziehen der Spritze mit Insulin ist besonders auf Blasenfreiheit zu achten. Die direkt sichtbare, aufgezogene Insulindosis ermöglicht dem Anwender eine leichte Kontrolle über die injizierte Insulinmenge. Für eine fehlerfreie Applikation erfordern Insulinspritzen dennoch Geschick und regelmäßige Anwendung.

Ein weiteres mittlerweile weltweit und insbesondere in Europa sehr verbreitetes Injektionsgerät ist der Insulinpen (Insulinstift).

Dieses schreibstiftgrosse Medizingerät wurde Mitte der 80er Jahre entwickelt und kommt hauptsächlich bei der intensivierten Insulintherapie zum Einsatz. Eine wesentliche Neuerung zu Insulinspritzen ist der Einsatz eines wechselbaren Medikamentenbehälters. Dieser Behälter, auch Karpulle bzw. Ampulle genannt, wird vom Hersteller mit dem Insulin befüllt ausgeliefert und vor Gebrauch in den Insulinpen eingesetzt. Bei Inbetriebnahme des Pens durchsticht eine Nadel die Dichtscheibe der Ampulle und realisiert bei der Applikation des Insulins die parenterale Injektion der vorgewählten Dosis. Ein Injektions- und Auslösemechanismus generiert während der Injektion einen Injektionshub, der den Vorschub eines Kolbens bzw. Stopfens in der Ampulle bewirkt und die Abgabe der vorgewählten Dosis in das Zielgewebe bedingt. Der Mechanismus besteht meist aus einer starren Kolbenstange mit einer dem Ampullenstopfenhub entsprechenden Baulänge.

Insulinpens werden in wegwerfbare ("disposable") und mehrfach verwendbare ("reusable") eingeteilt. Bei wegwerfbaren bilden die Ampulle und die Dosiermechanik eine vom Hersteller vorgefertigte Einheit und werden nach Entleerung der Ampulle gemeinsam entsorgt. Eine Wiederverwendung der Dosiermechanik ist nicht vorgesehen. Im Gegensatz zu den Fertigpens stellen mehrfach verwendbare Pens erhöhte Anforderungen an den Anwender. So muss bei Wechsel der Ampulle die Kolbenstange in die Startposition zurückgesetzt werden. Dies geschieht modellabhängig durch des Drehen bzw. Schieben der Kolbenstange bei gleichzeitiger Aktivierung einer Sonderfunktion in der Dosiermechanik. Dies muss der Anwender sehr sorgfältig durchführen, da aufgrund des täglichen Einsatzes und der hohen mechanischen Belastungen mitunter Fehlfunktionen, z. B. ein Verklemmen der Kolbenstange, auftreten können.

Mehrfach verwendbare Insulinpens werden weiterhin unterteilt in manuelle und halbautomatische Pens. Bei manuellen Pens betätigt der Anwender mit Fingerkraft den Injektionsknopf und bestimmt so Dauer und Verlauf der Injektion. Bei halbautomatischen Insulinpens hingegen wird vor Benutzung manuell eine Feder gespannt, die die notwendige Injektionsenergie speichert. Bei dem eigentlichen Injektionsvorgang wird die Feder vom Anwender entriegelt. Die Injektionsgeschwindigkeit ist durch die Federkraft festgelegt und kann nicht an persönliche Bedürfnisse angepasst werden.

In der DE 19 745 999 wird eine kompakte Schlauchpumpe besonders kleiner Bauweise offenbart. Diese Schlauchpumpe soll aus einem Förderkopf, einem Antrieb für den Förderkopf, einer Drehzahlregelung und anderen Bauteilen und Zubehör bestehen, wobei sich die Schlauchpumpe dadurch auszeichnet, dass der Pumpenkopf mit zugehörigem Antrieb auf einfache Weise aus dem Gehäuse entnommen werden kann und durch eine baugleiche, bauähnliche oder eine andere Baugruppe getauscht werden kann.

Ein großer Nachteil dieser Anordnung ist, dass der Pumpenkopf nur zusammen mit dem Antrieb aus dem Gehäuse entnommen werden kann. Dadurch wird das routinemäßige Auswechseln des Pumpenkopfes zur Aufrechterhaltung einer möglichst sauberen und keimarmen Behandlung teuer, schwerfällig und unpraktikabel.

In der US Patentanmeldung US 2002/0169439 A1 wird ein Gerät zur Abgabe von Flüssigkeit beschrieben, welches ein wegwerfbares Bauteil mit einem Ausgang, einer Energiequelle und einer Dosierungseinheit und ein wieder verwendbares Bauteil mit einer Kontrolleinheit beinhaltet. Das Gerät ist durch eine Fernbedienung kabellos steuerbar. Die Dosierungseinheit des wegwerfbaren Bauteils ist beispielsweise als peristaltische Pumpe ausführbar und befördert Flüssigkeit, die in einem Reservoir in dem Einweg-Bauteil enthalten ist, zum Ausgang. Dabei wird die Pumpe von einem Motor in dem wieder verwendbaren Bauteil angetrieben.

Die Erfindung betrifft eine Vorrichtung zum Bewegen von Flüssigkeiten, welche ein Arzneimittel in gelöster oder suspendierter Form enthalten, diese Vorrichtung bestehend unter anderem wenigstens aus
a) einem Motor;
b) einen Vorratsbehälter;
c) einem Pumpenkopf, der vom Motor aus a) angetrieben wird und mittels dessen die Flüssigkeit aus dem Vorratsbehälter abgeführt wird;
d) einer Steuerelektronik;
   gekennzeichnet dadurch, dass der Pumpenkopf mit lösbaren und wieder verbindbaren Schnittstellen zum Motor aus a) und dem Vorratsbehälter aus b) ausgestattet ist.

Eine Vorrichtung besteht aus einem oder mehreren miteinander verbundenen Bauteilen und dient der Ausführung eines bestimmten Zwecks. Der Zweck kann durch eine bestimmte Art des Gebrauchs festgelegt sein. Ein Zweck ist beispielsweise die Verwendung zur Injektion eines Arzneimittels, insbesondere die Injektionen von Insulin in den menschlichen oder tierischen Körper.

Die Erfindung besteht in einer bevorzugten Ausführungsform aus einer Vorrichtung wie vorstehend beschrieben, bei welcher der Pumpenkopf weiterhin mit einer lösbaren und wieder verbindbaren Schnittstelle zur Steuerelektronik ausgestattet ist.

Die Schnittstelle zwischen dem Pumpenkopf und dem Motor verbindet die beiden Teile funktionell. Bei dieser funktionellen Verbindung wird die Bewegung des Motors in Pumparbeit übergeführt. Der Motor kann hierfür in unterschiedlicher Weise mit Energie versorgt werden. Bevorzugt wird hierbei der Betrieb mittels einer Batterie (zum Einmalgebrauch oder wiederaufladbar) oder mittels Haushaltsstrom evtl. über einen zwischengeschalteten Adapter zur Anpassung der Spannung und/oder mittels Solarzellen. Die Pumparbeit dient zur Abführung von Flüssigkeit aus dem Vorratsbehälter. Dazu besteht eine Schnittstelle zwischen dem Pumpenkopf und dem Vorratsbehälter. Diese Schnittstelle ist so ausgelegt, dass die Flüssigkeitsbewegung zur Abfuhr der Flüssigkeit aus dem Vorratsbehälter durch entsprechende Bedienung oder Steuerung des Motors und/oder Pumpenkopfes gestartet, aufrechterhalten und gestoppt werden kann. In diese Schnittstellen sind Schläuche miteinbezogen. Die Verbindungen sollten fluiddicht ausgeführt sein. Weiter besteht eine Schnittstelle zwischen Pumpenkopf und Steuerelektronik. Diese Schnittstelle dient zur Übertragung von Sensordaten z.B. von einem Flusssensor, Temperatursensor, "Glukosesensor" oder anderen Sensoren, zur Steuerelektronik. Die Schnittstelle kann elektrisch, optisch, drahtlos ausgeführt sein. Die Steuerelektronik kann zur Aufrechterhaltung des Betriebszustandes des Geräts, der Koordination der verschiedenen Bestandteile, dem Austausch und der Verarbeitung von Betriebsdaten zwischen den verschiedenen Bauteilen, dem Austausch von Information und Input bezüglich des Benutzers oder der Überwachung der normalen Betriebsfunktionen und der Sicherheit bezüglich des Benutzers dienen.

Die Schnittstellen des Pumpenkopfes zum Motor, zum Vorratsbehälter und zur Steuerelektronik zeichnen sich dadurch aus, dass sie einfach und rasch gelöst und wieder verbunden werden können. Die Qualifizierung für einfaches Lösen und Verbinden erfolgt im Hinblick auf einen durchschnittlichen Bediener der Vorrichtung, der eine evtl. beigefügte Beschreibung vorher durchgelesen hat. Einfaches Lösen der Schnittstellen kann beispielsweise durch Auseinanderziehen der Teile, durch Drücken und nachfolgendes Drehen der Teile, durch umlagern eine Hebels, Schieben eines Schiebknopfes, oder Drücken eines Druckknopfes zur Entlastung aus einem Arretiermechanismus, weiterhin auch durch Abschrauben, Entkuppeln oder ähnliches Bewirkt werden. Einfaches Verbinden der Schnittstellen kann beispielsweise durch Drücken, Schieben, Ineinanderdrehen, Anschrauben, Ankoppeln, Anklicken, das Umlegen eines Hebels oder ähnliches bewirkt werden. Einfaches Lösen und Verbinden eine Schnittstelle liegt insbesondere vor, wenn Lösen und Verbinden nicht mit Hilfe eines Werkzeuges sondern allein durch Einsatz der Körperkraft eines Menschen (z.B. eines Patienten, Angehörigen des medizinischen Personals) insbesondere resultierend aus Bewegungen der Arme, Hände und/oder Finger erfolgt.

Die Erfindung besteht in einer bevorzugten Ausführungsform aus einer Vorrichtung wie zuvor beschrieben, bei welcher der Pumpenkopf nach Betrieb (d.h. Betätigung der Vorrichtung) gegen einen anderen Pumpenkopf ausgewechselt wird.

Der Pumpenkopf wird insbesondere jedes Mal, oder auch jedes zweite, dritte, vierte, fünfte, sechste, siebte, achte, neunte, zehnte Mal oder in längeren Intervallen jeweils nach Verwendung der Vorrichtung in einem Zyklus "Einschalten, Aufrechterhalten und Abschalten" des Pumpenkopfes zum Betrieb ausgewechselt. Der Wechsel des Pumpenkopfes erfolgt in jedem Fall, wenn die Pumpenleistung nachlässt. Insbesondere in den Anwendungsfällen, bei denen auf Sauberkeit und/oder möglichst geringe Anzahl an mikrobiologischen Keimen (beispielsweise bei medizinischen Behandlungen), Wert gelegt wird, ist der Pumpenkopf häufig d.h. jedes Mal oder jedes zweite Mal nach Gebrauch zu wechseln.

Die Erfindung besteht in einer weiteren bevorzugten Ausführungsform aus einer Vorrichtung wie zuvor beschrieben, bei welcher der Pumpenkopf eine Nadel trägt. Unter Nadel ist eine Injektionsnadel für den medizinischen Gebrauch zu verstehen. Eine Nadel umfasst eine Kanüle (meist aus Metall), durch welche Flüssigkeit oder Gas in/aus dem menschlichen oder tierischen Körper injiziert oder abgesaugt werden kann, sowie eine Haltevorrichtung, die über der Kanüle angebracht ist, mittels derer die Nadel an einer Spritze, einem Katheder, einer medizinischen Pumpe, einem Medikamentenstift (z.B. Insulinpen) oder einem anderen medizinischen Gerät befestigt werden kann. Die Nadel, welcher der Pumpenkopf trägt, dient insbesondere dazu, die aus dem Vorratsbehälter abgeleitete Flüssigkeit (z.B. Insulinzubereitung) in den menschlichen oder tierischen Körper zu injizieren.

Die Erfindung besteht in einer weiteren bevorzugten Ausführungsform aus einer Vorrichtung wie zuvor beschrieben, bei welcher der Motor aus einem Mikromotor besteht. Ein Mikromotor zeichnet sich durch klein bemessene Dimensionen aus. Seine Länge liegt zwischen 3 und 0,5 cm, seine Breite zwischen 0,5 und 1,5 cm und seine Höhe zwischen 0,5 und 1,5 cm. Ein Mikromotor zur Verwendung in der erfindungsgemäßen Vorrichtung beruht insbesondere auf einem elektromagnetischen Antrieb.

Die Erfindung besteht in einer weiteren bevorzugten Ausführungsform aus einer Vorrichtung wie zuvor beschrieben, bei welcher der Vorratsbehälter aus einer handelsüblichen Ampulle zur Aufnahme eines Medikaments besteht. Solche Ampullen werden für unterschiedliche Arzneimittel angeboten. Bekannt sind Ampullen (auch vial, Patrone oder Kartusche genannt), die Insulin in unterschiedlicher Art (z.B. langsam wirkendes wie Lantus oder schnell wirkendes wie Apidra oder normal wirkendes wie Insuman), Menge (z.B. 100 I.U., 200 I.U., 300 I.U., 500 I.U., 1000 I.U. oder andere Menge) als Lösung oder Suspension sowie als Mischung verschiedener Insuline enthalten. Insulinampullen (Insulinvial, Insulinpatrone, Insulinkartusche) werden mittels Spritzen und Insulinpens zur Injektion des Insulin in den menschlichen Körper bzw. mittels Insulinpumpen zur kontinuierlichen Zuführung des Insulin in den menschlichen Körper verwendet. Hersteller solcher Insulinampullen ist insbesondere die Firma Sanofi-Aventis. Die handelsübliche Versorgung mit Insulinampullen erfolgt in den meisten Ländern in der Regel über die Apotheken.

Die Erfindung besteht in einer weiteren bevorzugten Ausführungsform aus einer Vorrichtung wie zuvor beschrieben, bei welcher die Flüssigkeit Insulin enthält. Das Insulin kann in unterschiedlicher Art (z.B. langsam wirkendes wie Lantus oder schnell wirkendes wie Apidra oder normal wirkendes wie Insuman), Menge (z.B. 100 I.U., 200 I.U., 300 I.U., 500 I.U., 1000 I.U. oder andere Menge) als Lösung oder Suspension sowie als Mischung verschiedener Insuline vorliegen. Das Insulin kann tierischen Ursprungs oder gentechnologisch produziert sein.

Die Erfindung bezieht sich auf die Verwendung einer Vorrichtung in einer oder mehrerer der Ausführungsformen wie zuvor beschrieben zur Injektion eines Stoffes in den menschlichen oder tierischen Köper. Ein solcher Stoff ist insbesondere Insulin in Lösung oder als Suspension. Eine Injektion ist dabei insbesondere zu unterscheiden von der Zufuhr über eine Pumpe. Bei der Injektion wird der Stoff innerhalb einer kurzen Zeitspanne (z.B. 5 bis 60 Sekunden) mittels einer Spritze oder einem Medikamentenstift (z.B. Insulinpen) in aller Regel als vorher festgelegtes Gesamtvolumen in den Körper eingebracht. Der Medikamentenstift kommt nur während der unmittelbaren Injektion mit dem Körper in Kontakt. Die Zufuhr eines Stoffes über eine Medikamentenpumpe erfolgt über einen längeren Zeitraum (ab 60 Sek. bis zu mehreren Stunden) wobei die Medikamentenpumpe meist am Körper befestigt wird.

Die Erfindung bezieht sich weiterhin auf die Herstellung einer Vorrichtung in einer oder mehrerer der Ausführungsformen wie zuvor beschrieben, wobei
a) ein Motor bereitgestellt wird;
b) ein Vorratsbehälter bereitgestellt wird;
c) ein Pumpenkopf bereitgestellt wird;
d) die Einzelbestandteile gemäß a) bis c) zu einer funktionellen Einheit zusammengebaut werden.

Der Zusammenbau der erfindungsgemäßen Vorrichtung zu einer funktionellen Einheit bedeutet eine erfindungsgemäße Vorrichtung in einem betriebsbereiten Zustand herzustellen. Die funktionelle Einheit der erfindungsgemäßen Vorrichtung ist nach Inbetriebnahme insbesondere imstande, durch Wirkung des motorgetriebenen Pumpenkopfes Flüssigkeit aus dem Vorratsbehälter zu entnehmen. Diese entnommene Flüssigkeit kann in einer weiteren Verwendungsmöglichkeit der funktionellen Einheit durch diese in einem menschlichen oder tierischen Körper injiziert werden.

Die Erfindung bezieht sich auch auf eine medizinisches Gerät zur Injektion eines Arzneimittels in den menschlichen oder tierischen Körper, umfassend unter anderem
a) ein Gehäuse und/oder
b) ein Einstellmechanismus zur Voreinstellung einer durch das medizinische Gerät abzugebenden Menge des Arzneimittels und/oder
c) eine Anzeige und/oder
d) ein Auslösemechanismus zum Ingangsetzen und Durchführen der Injektion; gekennzeichnet dadurch, dass zusätzlich mindestens eine erfindungsgemäße Vorrichtung in einer oder mehrerer der Ausführungsformen wie zuvor beschrieben enthalten ist.

Ein medizinisches Gerät zur Injektion eines Arzneimittels ist insbesondere ein Pen (auch Stift genannt). Bekannt sind insbesondere Pens zur Injektion von Insulin (Insulinpen). Insulinpens sind in Apotheken erhältlich. Beispiele für gegenwärtig auf dem Markt befindliche Insulinpens sind der Opticlick, Optipen Pro, Optiset und Insulinpens anderer Hersteller.

Ein Gehäuse ist die äußere Hülle eines solchen medizinischen Geräts zu der je nach Ausführungsart auch eine Schutzkappe gehören kann. Das Gehäuse kann aus Kunststoff oder Metall gefertigt sein. Es hat in aller Regel eine längliche Form, enthält meist Aussparungen, Öffnungen oder Fenster, umfasst einen inneren Hohlraum und ist zur Aufnahme und Positionierung weiterer Bauteile geeignet.

Ein Einstellmechanismus ermöglicht die Voreinstellung einer später zu injizierenden Menge eines Arzneimittels. Der Einstellmechanismus kann mechanisch oder elektronisch betrieben werden. Der Einstellmechanismus ist so konstruiert, dass die voreingestellte Menge des Arzneimittels bis zur Durchführung der eigentlichen Injektion korrigiert werden kann.

Die Anzeige dient der Wiedergabe der voreingestellten und zu injizierenden Menge des Arzneimittels. Die Anzeige kann in mechanischer Art oder in Form einer LCD Anzeige erfolgen. Der Auslösemechanismus umfasst die vor Durchführung der eigentlichen Injektion eventuell erforderliche Entfernung von Luftblasen sowie den Start des Injektionsvorgangs bis zur Beendigung der Injektion durch entsprechende Ansteuerung des Motors und/oder des Pumpenkopfes. Das erfindungsgemässe medizinische Gerät kann eine zweite Anzeige oder weitere Anzeigen enthalten. Die erste oder die zweite oder eine weitere Anzeige können dazu verwendet werden, die Wiedergabe des aktuellen Gerätestatus bei der Injektion, z.B. verbleibende Restmenge, Temperatur, Glukosewert darzustellen. Eine solche Anzeige kann beispielsweise den Verlauf der Injektion mittels eines Fortschrittsbalkens darstellen

Ein medizinisches Gerät wie zuvor beschrieben umfasst in einer bevorzugten Ausführungsform mindestens ein Mittel zur Speicherung und/oder Verarbeitung von Daten und/oder Signalen sowie mindestens eine Schnittstelle zur Übertragung von Daten und/oder Signalen zu und/oder von einer externen technischen Einheit (bestehend beispielsweise aus einem PC, auf welchem ein Programm zur Speicherung und/oder Verarbeitung von Daten und/oder Signalen installiert ist, welche zur Speicherung und/oder Verarbeitung von Daten und/oder Signalen ausgelegt ist.

Ein medizinisches Gerät wie zuvor beschrieben ist insbesondere dadurch gekennzeichnet, dass es zur Injektion von Insulin, oder GLP-1 oder einem Heparin wie beispielsweise Lovenox besteht. Das Insulin kann ein lang wirkendes, ein kurz wirksames, ein Mischinsulin oder ein normal wirkendes Insulin tierischen oder menschlichen Ursprungs oder ein herkömmlich oder gentechnisch produziertes sein und als Lösung oder als Suspension vorliegen.

Das erfindungsgemäße medizinische Gerät in einer oder mehrerer seiner Ausführungsformen kann zur Prophylaxe und/oder Therapie einer Krankheit und/oder Fehlfunktion des Körpers mittels eines Stoffes, dessen pharmakologische Wirksamkeit im gastro-intestinalen Trakt abgeschwächt wird oder verloren geht, verwendet werden.

Das erfindungsgemäße medizinische Gerät in einer oder mehrerer seiner Ausführungsformen kann insbesondere zur Behandlung von Diabetes z.B. durch Verabreichung von GLP-1 verwendet werden.

Darüber hinaus kann das erfindungsgemäße medizinische Gerät in einer oder mehrerer seiner Ausführungsformen zur Verabreichung eines Peptid-Hormons (z.B. Glucagon, Thyroxin, Hypophysenhormon, Hypothalamushormon, Leptin u. a.) oder eines Wachstumshormons (z.B. Human Growth Hormone) verwendet werden.

Weiterhin kann das erfindungsgemäße medizinische Gerät in einer oder mehrerer seiner Ausführungsformen zur Verabreichung eines Heparins (z.B. niedermolekulares Heparin) und/oder von Lovenox verwendet werden.

Schließlich kann das erfindungsgemäße medizinische Gerät in einer oder mehrerer seiner Ausführungsformen zur Verabreichung eines Impfstoffes (z.B. Lebend- oder Totimpfstoff; Impfstoff zur Behandlung von Grippe, Masern, Mumps, Kinderlähmung, Tollwut, Wundstarrkrampf, Keuchhusten, Immunschwächekrankheit u. a.) und/oder zur Verabreichung von Antikörpern (z.B. monoklonale oder polyklonale Antikörper zur Behandlung einer bakteriellen oder viralen Infektion, Fehlfunktion des Immunsystems, Allergie, Krebs u. a.) verwendet werden.

Die Erfindung bezieht sich weiterhin auf die Herstellung eines medizinischen Geräts zur Injektion eines Arzneimittels in den menschlichen oder tierischen Körper, wobei
a) ein Gehäuse bereitgestellt wird;
b) ein Einstellmechanismus zur Voreinstellung einer durch das medizinische Gerät abzugebenden Menge eines Arzneimittels bereitgestellt wird;
c) eine Anzeige bereitgestellt wird;
d) ein Auslösemechanismus bereitgestellt wird;
e) evtl. elektronische Bestandteile bereitgestellt werden;
f) mindestens eine erfindungsgemäße Vorrichtung in einer oder mehrerer der Ausführungsformen wie zuvor beschrieben bereitgestellt wird;
g) die Einzelbestandteile aus a) bis f) zu einer funktionellen Einheit zusammengebaut werden.

Eine Vorrichtung besteht aus einem oder mehreren Bauteilen und dient der Ausführung eines bestimmten medizinischen Zwecks insbesondere der Injektion eines Stoffes in den menschlichen oder tierischen Körper. Ein Bauteil besteht aus einer oder mehr als einer Komponente und dient der Erfüllung einer technischen oder nicht technischen Funktion. Eine Funktion ist technisch, wenn dabei eine Übertragung von Kraft, Arbeit, Energie, Material (Stoff), Daten und/oder Signalen, die Aufrechterhaltung der Struktur und/oder Form oder die Lagerung eines Stoffes bzw. Speicherung von Informationen betroffen ist. Eine Funktion ist nicht technisch, wenn die Eingabe oder Ausgabe von Information von von oder an den Benutzer der Vorrichtung oder eines Stoffes von oder an den Benutzer der Vorrichtung betroffen ist.

Ein Bauteil kann beispielsweise Teil eines technischen Geräts sein, welches eine Teilfunktion im Verhältnis zur Gesamtfunktion des Geräts zur Verfügung stellt. Ein Bauteil ist beispielsweise ein Vorratsbehälter. Vorratsbehälter kann eine auswechselbare Ampulle enthaltend einen Stoff (insbesondere ein Medikament wie z. B. Insulin) sein. Die auswechselbare Ampulle kann sich insbesondere zur Verwendung in einem Insulinpen oder einer anderen Vorrichtung zur Injektion eines Medikaments in den menschlichen oder tierischen Körper eignen. Ein anderes Beispiel für ein technisches Bauteil ist eine Vorrichtung zum Pumpen oder eine Pumpe. Weitere Beispiele für technische Bauteile sind insbesondere Spritzen, Nadeln, Kolbenstangen, Dosiereinrichtungen, mechanische Anzeigen, Schläuche, Dichtungen, Batterien, Motoren, Getriebe, elektronische Anzeigen, elektronische Speicher oder elektronische Steuerungen. Als Zweck im Zusammenhang mit der technischen Vorrichtung soll insbesondere die Bewegung von Flüssigkeit von einem Ort zu einem anderen verstanden werden. Ein Zweck ist beispielsweise durch Bewegung eines Flüssigkeitsvolumens von einem Vorratsbehälter zu einer Ableitung definiert. Zweck kann auch die Injektion eines Medikaments in den menschlichen oder tierischen Körper sein.

Ein Bauteil kann mit einem oder mehreren anderen Bauteilen in technischer Weise verbunden sein, um gemeinsam einen Zweck zu erfüllen. Eine technische Verbindung ist beispielsweise eine Verbindung von Bauteilen, die sich zur Übertragung von Kraft, Arbeit, Energie, Material (Stoff), Daten und/oder Signalen eignet. Verbunden werden können die Bauteile z. B. über eine mechanische Kupplung, eine feste mechanische Verbindung (Kleben, Schrauben, Nieten, Gestänge oder ähnliches), ein Zahnrad, eine Klinke, eine Sperre, einen metallischen Draht, ein Lichtleiter, eine Funkverbindung, ein elektromagnetisches Feld, einen Lichtstrahl oder ähnliches.

Injektion ist die Einbringung von Stoffen insbesondere von Flüssigkeiten mittels einer Kanüle samt Spritze oder funktionell vergleichbarer Vorrichtung wie insbesondere einen Pen in den menschlichen oder tierischen Körper. Man kennt unter anderem subkutane, intramuskuläre, intravenöse, intrakutane und intraartikuläre Injektion. Die subkutane Injektion erfolgt unter die Haut, sie ist relativ einfach auszuführen, wenig schmerzhaft und kann vom Patienten selbst vorgenommen werden. Die intramuskuläre Injektion erfolgt in den Muskel. Da hierbei größere Risiken bestehen, wie beispielsweise die schmerzhafte Verletzung von Knochenhaut, wird diese meist von medizinischem Personal vorgenommen. Die intravenöse Injektion erfolgt nach Venenpunktion direkt über eine Vene.
Bei der intrakutanen Injektion wird ein Arzneimittel direkt unter die Lederhaut verbracht. Bei der intraartikulären Injektion wird eine Flüssigkeit in ein Gelenk injiziert.
Die Injektion eines Stoffes in den menschlichen oder tierischen Körper ist insbesondere zu unterscheiden von der Einbringung eines Stoffes durch eine Medikamentenpumpe, eine Infusion oder eine andere Art der kontinuierlichen und über einen gewissen Zeitraum erfolgenden Zuführung.

Eine Kanüle ist im Wesentlichen eine hohle Nadel, die gewöhnlich aus Metall (z. B. Stahl, Edelstahl, Gold, Silber, Platin) gefertigt ist. Das Ende der Kanüle ist häufig mit einem schrägen Schliff geschärft. Die Kanüle kann an einem Ende spitz und/oder geschärft und am anderen Ende stumpf sein, sie kann aber auch an beiden Enden spitz und/oder geschärft sein. Die Kanüle trägt an einem der beiden Enden einen meist konusförmigen Aufsatz aus beispielsweise Kunststoff mittels dessen ein Anbringen der hohlen Nadel zum Beispiel durch Aufstecken oder Aufschrauben an einem medizinischen Gerät wie beispielsweise einer Spritze, einem Medikamentenpen insbesondere einem Insulinpen, einem Medikamentenbehälter oder einer Medikamentenpumpe möglich ist. Die Kanüle dient in der funktionellen Interaktion mit einer Spritze, einem Pen, einer Pumpe oder einem anderen dafür geeigneten medizinischen Gerät der Entnahme oder Zufuhr einer Flüssigkeit aus oder in den menschlichen oder tierischen Körper.

Die Angabe des Kanülendurchmessers (Außendurchmesser) erfolgt meist in mm oder in Gauge (18 Gauge = 1,2 mm; 20 Gauge = 0,9 mm; 21 Gauge = 0,8 mm; 22 Gauge = 0,7 mm; 23 Gauge = 0,6 mm; 25 Gauge = 0,5 mm; 27 Gauge = 0,4 mm). Eine andere Kenngröße zur Charakterisierung der Kanüle ist deren Länge. Typische Längen von Kanülen sind 40 mm, 30 mm, 25 mm, 8 mm, 6 mm und andere Längen.

Ein medizinisches Gerät ist insbesondere ein Gerät zur Injektion des Stoffes in den menschlichen oder tierischen Körper. Neben einer Spritze kann ein solches Gerät zur Injektion ein Medikamentenpen wie beispielsweise ein Insulinpen sein. Medikamentenpens sind in unterschiedlicher Form und für unterschiedliche Zwecke geeignet und von verschiedenen Herstellern auf dem Markt erhältlich (z. B. Optiklick, Optipen, Optiset).

Jeder Insulinpen muss zahlreichen Anforderungen hinsichtlich der Bedienfreundlichkeit genügen, um die sichere und fehlerfreie Anwendung zu ermöglichen. Grundvorrausetzung ist das Anzeigen der vorgewählten Dosis bzw. der Restmenge in der Ampulle. Weiterhin ist die Dosiseinstellung sowie der Abschluss des Injektionsvorgangs hörbar, fühlbar und sichtbar zu gestalten. Diese Sicherheitsanforderung ergibt sich vor allem aus den eingeschränkten Wahrnehmungsmöglichkeiten bei älteren Diabetes Typ-2 Patienten.

Neben nadel behafteten Insulinpens kommen auch nadelfreie Injektionssysteme bei der Insulintherapie zum Einsatz. Ein aktuelles Anwendungsbeispiel für nadelfreie Injektionssysteme ist das Injektionssystem Injex der Fa. Rösch AG. Bei diesem Injektor wird das Insulin mit extrem hohem Druck durch eine Mikronadel in die Fettschicht der Haut geschossen. Eine vor der Injektion manuell gespannte Sprungfeder speichert die dafür notwendige Injektionsenergie. Das Injektat wird dabei homogen und konusförmig im Fettgewebe verteilt.

Ein nicht zu vernachlässigender Vorteil dieser Geräte ist die nadelfreie Injektion des Medikaments, die bei einigen Patienten die psychologische Hemmschwelle der Insulinapplikation herabsetzt. Ferner schließt die nadelfreie Injektion eine Infektion der Einstichstelle aus. Nachteilig gegenüber herkömmlichen Insulinpens erweist sich das Umfüllen des Insulins in spezielle Ampullen, die vergleichbar größere Masse des Geräts sowie das Mitführen weiteren Zubehörs zum Spannen der Feder.

Im Unterschied zu Insulinspritzen sind Insulinpumpen vollautomatische Infusionssysteme zur kontinuierlichen subkutanen Insulininjektion. Sie haben etwa die Größe einer Zigarettenschachtel und werden permanent am Körper getragen. Das kurzwirkende Insulin wird über einen Katheder und einer in der Haut liegenden Nadel nach dem vom Patienten vorgegebenen Programm in das Hautgewebe gespritzt. Die Aufgabe der Insulinpumpe ist es, den kontinuierlichen Insulinausstoß der Bauchspeicheldrüse zur Senkung des Blutzuckerspiegels zu imitieren, ohne aber einen geschlossenen Regelkreis der Blutzuckerregulierung realisieren zu können. Aufgrund der kontinuierlichen und anpassbaren Zuführung des Insulins bieten diese Pumpen Vorteile vor allem für sportlich aktive Menschen bzw. solche mit stark wechselnden Tagesabläufen. Mit der Insulinpumpentherapie können starke Schwankungen des Blutzuckers, z. B. bei Diabetikern mit ausgeprägtem DAWN-Phänomen, ausgeglichen werden, die mit herkömmlichen Methoden nur mit erhöhtem Aufwand beherrschbar sind. Ein Nachteil ist, dass bei unterbrochener Insulinzufuhr aufgrund des fehlenden Insulinvorrats im menschlichen Körper schwere Stoffwechselentgleisung auftreten können. Insulinpumpen gibt es in verschiedenen technischen Ausführungen, wobei sich Geräte mit spritzenartigen Behältern im Laufe der technischen Entwicklung durchgesetzt haben. Analog zu den Insulinpens mit Nadeln befindet sich das Insulin in einem Vorratsbehälter mit beweglichem Stopfen. Dieser wird durch eine motorisch angetriebene Kolbenstange bewegt.

Aufgrund der vollautomatischen und kontinuierlichen Insulinabgabe sind die Pumpen mit einer Vielzahl von Sicherungssystemen versehen, um den Anwender vor folgenschweren Fehlfunktionen zu schützen. Dies befreit aber nicht von einer eigenverantwortlichen und vorrausschauenden Nutzung des Gerätes.

Auf Basis der heutigen Injektionsgeräte und der technologischen Weiterentwicklung in der Medizin- und Mirkosystemtechnik ist ein Trend zu vollautomatischen miniaturisierten Medikamentendosierungssystemen zu verzeichnen. Die weitere Entwicklung könnte in Richtung implantierbarer und extrakorporaler Medikamentendosiersysteme verlaufen. Mit implantierbaren Insulinpumpen verfolgt man das Ziel, den Diabetiker vom täglichen Spritzen des Insulins zu befreien, ohne dabei ein externes Gerät am Körper tragen zu müssen.

Insulinpens sind in den wesentlichen ergonomischen und sicherheitstechnischen Merkmalen in der Norm EN ISO 11608 konzentrieren. Diese schließt ebenfalls die geometrisch-stofflichen Eigenschaften der Insulinampullen und Pennadeln ein. Somit ist für den Benutzer die Handhabung und die Bedienung eines Pens weitgehend einheitlich und vom Modell unabhängig.

Auf den Inhalt der Norm EN ISO 11608, soweit sich dieser auf Insulinpens, Insulinampullen sowie Nadeln bezieht, wird hiermit ausdrücklich als Bestandteil vorliegender Offenbarung verwiesen.

In der konstruktiven Ausführung der Pens lassen sich zum Teil beachtliche Unterschiede bei den Pens der verschiedenen Hersteller feststellen. Diese sind beispielsweise mit der Bestimmung für unterschiedliche Zielgruppen (Kinder, ältere Menschen) begründet. Aufgrund der Anforderungen aus der Norm EN ISO 11608 beschränken sich die Unterschiede vor allem auf den Injektionsmechanismus und den Auslösemechanismus. Der Dosiswähler und die Dosieranzeige unterliegen meist ergonomischen Anforderungen und ergeben sich aus den konstruktiven Allgemeinbedingungen des jeweiligen Modells.
Das wesentliche Funktionselement eines Insulinpens ist der Injektionsmechanismus. Er bestimmt die Bauform und Baugröße des Pens sowie die konstruktive Ausführung des Auslösemechanismus und des Dosiswählers. Der Mechanismus übersetzt die am Dosiswähler voreingestellte Dosis mit der vom Auslösemechanismus kommenden Injektionsenergie in einen Injektionshub des Stopfens in der Ampulle. Diese Energie wird entweder direkt auf den Injektionsmechanismus oder durch bewegungsumformende Getriebe übertragen.

Der Injektionsmechanismus in Gestalt der Kolbenstange ist technisch in vielfältiger Form realisierbar.
Bei derzeitig auf dem Markt verfügbaren Insulinpens haben sich Lösungen mit einer starren (z. B. Gewindespindel, Zahnstange) oder einer flexiblen (z. B. gebogenen Zahnstange, gebogenen Druckfeder) Bauform etabliert. Andere mögliche Ausführungen wie teleskopischer Kolbenstange (z. B. Schraubengetriebe, Zugmittelgetriebe, Druckmittelgetriebe, Koppelgetriebe) kommen bei den gegenwärtig im Handel verfügbaren Insulinpens nicht zum Einsatz.

Die konstruktiven Lösungen der starren und flexiblen Bauform sind sehr unterschiedlich und vom Typ des Pens, d. h. wieder verwendbarer Pen oder Fertigpen, abhängig. Als Kolbenstangen kommen Gewindespindeln oder Zahnstangen bzw.
Kombinationen beider zum Einsatz. Beim Dosierwähler wird mit Hilfe von Rastvorrichtungen ein der Dosis entsprechender Drehwinkel voreingestellt und mit nachgeschalteten Schrauben- und Zahnradgetrieben auf den Injektionsmechanismus übertragen sowie in den Injektionshub transformiert.

Die Abgabe des Medikaments erfolgt durch Vorgabe eines Injektionshubs und der resultierenden Verschiebung des Stopfens. Die abgegebene Flüssigkeitsmenge ist vom Injektionshub und dem Innendurchmesser der Ampulle abhängig. Zur Vermeidung von Dosierfehlern sind entsprechend Herstellervorgaben und der Norm EN ISO 11608 Luftblasen vollständig zu entfernen. Weiterhin ist nach Abgabe der Flüssigkeit eine hinreichend lange Wartezeit einzuhalten, um einen stationären Zustand, d. h. Normaldruck der Flüssigkeit und Relaxation des Stopfens in der Ampulle zu gewährleisten.

Der Vorratsbehälter für das Medikament (auch als Ampulle oder Kartusche bezeichnet) beeinflusst den Aufbau und die Funktionsstruktur des Medikamentenstifts. Man kann hierbei als Teilfunktionen unterschieden einmal eine Schutzfunktion für das Medikament, dann eine Förderfunktion und schließlich eine Kopplungsfunktion zum Injektionssystem des Medikamentenstiftes. Die Schutzfunktion wird durch die Ampulle insgesamt, d. h. durch Stopfen, Glaskörper und Dichtscheibe realisiert. Die Förderfunktion für das Medikament vermittelt der Stopfen, welcher mit Hilfe des Injektionsmechanismus verschoben wird und eine Volumenänderung in der Ampulle bewirkt. Die Kopplungsfunktion zum Injektionssystem schließlich wird durch Dichtmittel (z. B. Dichtscheibe) hergestellt.

Bei einem automatischen Medikamentenstift (z. B. automatischer Insulinstift oder Insulinpen) wird die Injektionsenergie von einem Antrieb mit nachgeordnetem Getriebe aufgebracht. Zusätzlich sind Energieversorgung und Steuereinrichtung notwendig.

Im erfindungsgemäßen Injektionsmechanismus erfolgt die Förderung des Medikaments (z. B. durch Insulin) nicht über die Verschiebung des Stopfens mittels eines Injektionsmechanismus sondern über die Einführung einer Pumpvorrichtung. Die Pumpvorrichtung wird zwischen Ampulle und Injektionssystem eingefügt und ist mit entsprechenden Schnittstellen zu versehen.
Die Pumpvorrichtung kann mit einer Flusssensorik versehen werden. Sie steht in direktem Kontakt zum Medikament z. B. Insulin, woraus sich zusätzliche
Anforderungen wie verminderte Keimzahl, Sterilität, Biskompatibilität u. a. ergeben können.
Bei Anwendung dieses Funktionsprinzips ändern sich im Vergleich zu einem herkömmlichen Medikamentenstift zur Injektion (z. B. einen Insulinpen) zahlreiche Zustandsgrößen z. B. der (Flüssigkeitsdruck im Medikamentenbehälter) da beim Heraussaugen des Medikaments ein Unterdruck entsteht.

Insulinampullen dienen als Primärpackmittel für das Medikament und müssen hohe Standards erfüllen. Dies betrifft die Maßhaltigkeit der Ampulle im Hinblick auf die Dosiergenauigkeit und Kompatibilität mit anderen Komponenten. Die Norm EN ISO 11608-3 greift diese Forderungen auf und beschreibt die grundlegenden Gesichtspunkte und den geometrisch-stofflichen Aufbau, ohne die Ampullengestaltung unnötig einzuschränken. Ebenso muss die pharmazeutische Dichtigkeit der Ampulle gewährleistet sein.

Die Ampullen bestehen aus mehreren Teilkomponenten. Die wichtigste ist der Zylinder aus pharmazeutischem Glas mit einer hohen Neutralität und chemischen Beständigkeit gegenüber dem Insulin. Vor der Befüllung wird die Oberflächenqualität des Zylinders durch Silikonisierung verbessert. Diese Oberflächenvergütung verringert die Gleit- und Losbrechkräfte des Stopfens, erhöht die Dosiergenauigkeit und verringert das Herauslösen von Glasbestandteilen bei langer Lagerzeit. Der Grad der Silikonisierung korreliert dabei mit der Höhe der Reibkräfte des Stopfens, wobei ein Grenzwert durch die Sensibilität des Insulins gegenüber dem Silikon gesetzt wird.

Die Ampulle wird beiderseitig durch elastomere Verschlussteile, den Stopfen und die Dichtscheibe, abgedichtet. Entscheidend sind dabei die nachgewiesene mechanische Dichtigkeit bei verschiedenen Drucksituationen sowie die mikrobiologische Dichtigkeit gegenüber Keimen in Langzeitversuchen. Wichtig sind weiterhin die maximal vertretbaren Stopfenkräfte und die Anzahl von Durchstichen der Dichtscheibe mit einer Kanüle.

Pennadeln sind sterile Einwegprodukte, die zum Einsatz kommen, um das Insulin aus der Ampulle in das Zielgewebe zu leiten. Sie unterliegen ebenso wie Ampullen strengen Anforderungen, da erst durch das Zusammenspiel beider Komponenten die eigentliche Funktionalität des Insulinpens erzielt wird. Die Nadel besteht aus einer beiderseitig geschliffenen Kanüle, die in einem Ampullenansatzstück eingefasst ist. Optimierte Kanülenschliffe ermöglichen dem Patienten ein weitgehend schmerzfreies Einstechen in das Zielgewebe und verursachen beim Wiederrherausziehen nur geringe Gewebeschädigungen. Ebenso wird die Ampullendichtscheibe ohne starke Fragmentation durchbohrt. Dies ist eine zwingende Vorraussetzung, da auch bei regelmäßigem Wechsel der Nadel die Dichtigkeit der Ampulle gewährleistet werden muss. Das Ampullenansatzstück sorgt für einen festen Sitz auf dem Insulinpen.

Auch wenn Pennadeln nach zwei- bzw. mehrmaligem Gebrauch für das Auge kaum sichtbare Abnutzungserscheinungen aufweisen, sollten sie dennoch nach jeder Injektion aus Gründen der Sterilität gewechselt werden. Zudem kann auskristallisiertes Insulin die Nadel verstopfen. Fernerhin gelangt bei Temperaturschwankungen Luft in die Ampulle, die gleichermaßen Dosierfehler verursacht. So bewirkt bereits ein Temperaturwechsel von 15 K, dass bis zu 15 µl Luft in die Ampulle eintreten.

Die Mikrofluidik ist ein Teilgebiet der Mikrosystemtechnik und umfasst Entwurf, Herstellung, Anwendung und Untersuchung von Mikrosystemen, die Fluidmengen in Kanalquerschnitten mit Abmessungen von 1 µm bis 1 mm manipulieren und behandeln. Mikrofluidische Systeme kommen in der Medizintechnik, der Biochemie, der chemischen Verfahrenstechnik und Analytik sowie der Mirkoreaktionstechnik zum Einsatz. Diese Mikrosysteme können dabei Abmessungen im Millimeter- und Zentimeterbereich haben, da für praktische Anwendung die Fluidmenge und nicht die Größe des mikrofluidischen Systems von Bedeutung ist. Zudem weisen solche Systeme aufgrund geringer Fluidmengen und oftmals kleiner Systemgrößen bedeutende Unterschiede gegenüber konventionellen fluidischen Systemen auf. Mit der Miniaturisierung ändert sich das Verhalten der Fluidströmung, da oberflächengebundene Effekte sowie elektrostatische und elektrokinetische Kräfte dominieren. daher sind neue Herangehensweisen für Entwurf, Herstellung und Charakterisierung von mikrofluidischen Komponenten, z. B. Mikropumpen und Sensoren, notwendig. Infolge konstanter Energiedichte der Aktuatoren sinkt deren Abgabeleistung, sodass diese nicht mit der von herkömmlichen Komponenten im Makrobereich vergleichbar sind. Aus diesem Grund werden häufig externe Aktuatoren eingesetzt, welche die Abmessungen des Gesamtsystems mitunter erheblich vergrößern. Weiterhin begrenzen Physik und Chemie der zu transportierenden Teilchen und Moleküle die Miniaturisierung mikrofluidischer Komponenten.

Diabetes mellitus ist eine Erkrankung, bei der der Körper selbst keine oder nicht mehr ausreichende Mengen an Insulin produzieren bzw. adäquat verwenden kann. Insulin wird benötigt, um Zucker aus dem Blut in die Körperzellen zu transportieren. Dar Blutzuckerspiegel wird ständig in engen Grenzen konstant gehalten (60-100 mg % bzw. 3,33-5,55 mmol/l). Dies erfolgt durch das Zusammenspiel der beiden Hormone Insulin und Glucagon.

Die Diagnose von Diabetes mellitus erfolgt nach Blutentnahme mittels entsprechender Laborgeräte. Es muss mindestens zweimal zu unterschiedlichen Zeitpunkten ein erhöhter Blutzuckerwert nachgewiesen werden, um die Diagnose zu erhärten.

Von Diabetes mellitus spricht man, wenn der Glukosewert gemessen im Blutplasma in wenigstens einem der angeführten Fälle den angegebenen Wert übersteigt:
a) Nüchternblutzucker - 7,0 mmol/l, bzw. 126 mg/dl
b) Blutzucker zwei Stunden nach Gabe von 75 mg Glukose (oraler Glukose-Toleranztest) - 11,1 mmol/l bzw. 200 mg/dl
c) Blutzucker 11,1 mmol/l bzw. 200 mg/dl verbunden mit starkem Durst (Polydipsie), häufigem Wasserlassen (Polyurie) oder Gewichtsverlust.

Unbehandelt führt Diabetes zu erhöhten Blutzuckerwerten, die zu verschiedenen Symptomen und Spätfolgen führen können wie beispielsweise Polyneuropathie, Mikroangiopathie, Makroangiopathie, Retinopathie, Nephropathie und anderen. Das Risiko von diabetischen Spätschäden ist umso geringer, je niedriger die nichtenzymatische Glykierung der Erythrozyten (HbA1c-Wert) ist

Diabetisches Koma ist eine lebensgefährliche Akutkomplikation von Diabetes. Der Blutzuckerwert kann dabei über 1000 mg/dl erreichen einhergehend mit einer starken Übersäuerung des Blutes (metabolische Azidose). Diabetisches Koma kann unter anderem ausgelöst werden durch Infekte, Aufnahme von zu viel Kohlehydraten, Alkoholmissbrauch oder falsche Dosierung des Insulin.

Man unterscheidet den Typ 1 Diabetes von Typ 2 Diabetes. Beim Typ 1 Diabetes liegt von Anfang an ein absoluter Insulinmangel vor, der nur mit Insulingabe behandelt werden kann.
Der Typ 2 Diabetes ist gekennzeichnet durch eine verminderte Insulinempfindlichkeit und einen relativen Insulinmangel. Typ 2 Diabetes lässt sich meist zunächst mit diätetischen Maßnahmen und Tabletten behandeln. Häufig wird im Verlauf der Erkrankung eine Insulin-Substitution nötig.

Der Typ 2 Diabetes ist vorwiegend in den industrialisierten Ländern eine weit verbreitete Krankheit geworden. Als Hauptursache gelten Überernährung, Bewegungsmangel und Übergewicht. Dem Typ 2 Diabetes lässt sich durch Bewegungs-Training und diabetische Maßnahmen, insbesondere auf Gewichtsreduktion abzielend, wirksam entgegenwirken. Daneben können im Falle des Typ 2 Diabetes orale Antidiabetika wie z. B. Acarbose, Biguanide, Sulfonylharnstoff, Glitazon und andere eingesetzt werden. Die Therapie, unter Verwendung von Insulin wird erforderlich, wenn mittels der genannten Maßnahmen der Blutzuckerspiegel nicht mehr mit ausreichender Nachhaltigkeit im oder nahe dem Normbereich gehalten werden kann.

Zur Insulintherapie stehen verschiedene Insuline zur Verfügung. Man unterscheidet gewöhnlich nach Wirkdauer oder chemischer Struktur. Ein Analoginsulin weist im Vergleich zu Humaninsulin an einzelnen Positionen unterschiedliche Aminosäuren auf. Dadurch können sich die Eigenschaften ändern.

Zu den schnellwirksamen Insulinen rechnet man das Humaninsulin sowie verschiedene schnell und kurz wirksame Insulin-Analoga wie Glulisin (Handelsname: Apidra), Lispro (Handelsname: Humalog) und Aspart (Handelsname: Novo Rapid).

Langsam wirkende oder Verzögerungsinsuline sind das NPH-Insulin (durch Neutral Protamin Hagedorn verzögert wirkendes Humaninsulin), Zinkinsuline und verschiedenen Insulin-Analoga wie Glargin (Handelsname: Lantus) und Detemir (Handelsname: Levemir).

In der Insulintherapie werden außerdem Mischinsuline und neuerdings inhalative Insuline verwendet.

Mischinsuline bestehen aus einem schnellwirksamen Insulin und einem Verzögerungsinsulin in verschiedenen Mischungsverhältnissen. Üblich sind Mischungen von 10/90 %, 25/75 %, 30/70 %, 50/50 %. Eine Insulintherapie muss immer von regelmäßigen Bestimmungen des Blutzuckerspiegels begleitet werden.

Bei der konventionellen Insulintherapie wird zu festgesetzten Zeiten eine bestimmte Menge Mischinsulin gespritzt. Die intensivierte konventionelle Insulintherapie kommt überwiegend bei der Therapie von Typ-1-Diabetikern zum Einsatz. Hierbei wird eine Grundversorgung über ein Verzögerungsinsulin (Basis) gewährleistet und zusätzlich zu den Mahlzeiten ein schnellwirksames Insulin (Bolus) gegeben.

Die kontinuierliche subkutane Insulininfusion mittels einer Pumpe kommt überwiegend für Typ-1-Diabetiker in Frage. Das Insulin wird nicht gespritzt sondern von einer kleinen Pumpe in den Körper geleitet. Die Pumpe befindet sich dauernd am Körper. Das Insulin wird über einen Katheter mit Kanüle zugeführt. Die Insulinpumpe gibt gewöhnlich schnell wirkendes Insulin in kleinen gleichmäßigen Abständen über einen längeren Zeitraum ab.

Glucagon-like-Reptid 1 (GLP1) zählt neben Glucose-dependant insulinotropic peptid (GIP) zu den wichtigsten Vertretern der Inkretine. Inkretine werden als Hormone im Darm gebildet und regulieren unter anderem den Blutzuckerspiegel durch Anregung der Insulin-Ausschüttung im Pankreas.

Die Menge der gebildeten Darmhormone ist abhängig von der oral aufgenommenen Menge an Kohlenhydraten. Der Spiegel an GLP1 steigt nach oraler Glukose-Aufnahme sehr viel stärker als nach der intravenösen Gabe von Glukose. Mit Untersuchungen konnte gezeigt werden, dass die intravenöse Infusion und die subkutane Injektion von GLP1 bei Typ 2-Diabetikern in vielen Fällen zu einer kompletten Normalisierung des Blutzuckerspiegels führt. Problematisch ist, dass GLP1 innerhalb sehr kurzer Zeit durch Dipeptidylpeptidase IV (DPP-IV) inhibiert wird. Eine subkutane Injektion von GLP1 kann nur über ca. 1-2 Stunden wirksame Plasmakonzentrationen aufrechterhalten. Eine Lösung in Richtung nachhaltiger Wirkung von GLP1 könnte bei der Entwicklung länger wirksamer GLP-Analoga oder auch der Hemmung von DPP-IV durch Arzneimittel zu finden sein.

Wachstumshormone sind Substanzen, die bei Menschen, Tieren und Pflanzen das Wachstum stimulieren. Man kennt beispielsweise das Somatotropin (Mensch), das bovine Somatotropin (Rind) und Auxin sowie Gibberellsäure (Pflanze).

Somatotropin (STH) ist auch unter den Bezeichnungen Human Growth Hormone (HGH), Growth Hormone (GH) oder Wachstumshormon (WH) bekannt. STH ist ein Peptidhormon mit 191 Aminosäuren. Die Bildung erfolgt im Hypophysenvorderlappen unter Regulierung des Somatotropin-releasing-Faktor (SRF; GHRH; GRF) aus dem Hypothalamus. STH ist für ein normales Längenwachstum unbedingt erforderlich. Bei verminderter Produktion oder einem verminderten Ansprechen der Zellen auf STH kommt es zu einem Kleinwuchs. Bei einer Überproduktion kommt es zu Riesenwuchs bzw. Akromegalie.

Kleinwuchs, der durch Mangel am Wachstumshormon verursacht ist, wird seit einigen Jahren durch Gabe von STH behandelt. Die Gewinnung erfolgte zuerst aus Hypophysen von Toten, bevor das STH seit 1985 gentechnologisch hergestellt werden konnte.

Interferone werden als Gewebehormone von menschlichen oder tierischen Leukozyten, Fibroblasten oder T-Lymphozyten gebildet. Ein Interferon ist ein Protein oder Glykoprotein mit einer immunstimulierenden (z. B. antiviral) oder antihormonen Wirkung. Man unterteilt die Interferone in Alpha-Interferone, Beta-Interferone und Gamma-Interferone. Interferone sind von verschiedenen Herstellern gegen Indikationen wie Viruserkrankungen (z. B. SARS), Krebs, multiple Sklerose, Hepatitis B/C, Hepatitis C erhältlich.

Bei einem Impfstoff handelt es sich um eine biologisch oder gentechnisch hergestellte Zusammensetzung enthaltend unter anderem einzelne Proteine und/oder RNA bzw. DNA Bruchstücken und/oder abgetötete oder abgeschwächte Erreger (z. B. Influenza, SARS, Pockervirus, Erreger von Masern, Mumps, Röteln, Kinderlähmung, Erreger des Keuchhustens).

Man kennt Lebendimpfstoffe (z. B. Kuhpocken), attenuierte Lebendimpfstoffe mit abgeschwächten Viren oder Bakterien (z. B. MMR-Impfstoff; Gelbfieber, Kinderlähmung) und Totimpfstoffe mit inaktivierten oder abgetöteten Viren oder Bakterien bzw. deren Bestandteilen (z. B. Influenza, Cholera, Beulenpest, Hepatitis A).

Heparine sind therapeutisch eingesetzte Substanzen zur Hemmung der Blutgerinnung. Heparine bestehen aus jeweils abwechselnden Folgen von D-Glucosamin und D-Glucuronsäure bzw. L-Iduronsäure. Kettenlängen bestehend aus 5 Einheiten können bereits gerinnungshemmend sein.

Die Polysaccharidketten haben meist ein Molekülgewicht zwischen 4000 und 40000. Neben unfraktionierten Heparinen kommen auch niedermolekulare fraktionierte Heparine mit einem Molekulargewicht von ca. 5000 zum Einsatz. Heparine werden nicht aus dem Magen-Darm-Trakt resorbiert, sonder müssen parenteral appliziert werden. Heparine wirken über die Bindung an Antitrhombin III und eine dadurch beschleunigte Inaktivierung von aktivierten Gerinnungsfaktoren.

Lovenox (auch bekannt als Clexane) ist eine kommerziell erhältliche Arzneimittelzubereitung mit dem pharmakologisch aktiven Wirkstoff Enoxaprin-Natrium. Der Wirkstoff zählt zu den niedermolekularen Heparinen mit einer linearen Dosis-Wirkungs-Beziehung und einer konstant hohen Bioverfügbarkeit.

Indikationsgebiete von Lovenox sind die Primärprohylaxe tiefer Venenthrombosen, die Therapie tiefer Venenthrombosen mit und ohne Lungenembolie, die Therapie von instabiler Angina pectoris und des so genannten Nicht-Q-Wellen Herzinfarkts sowie der Thromboseprophylaxe und Gerinnungshemmung während der Hämodialyse.

### Beispiele

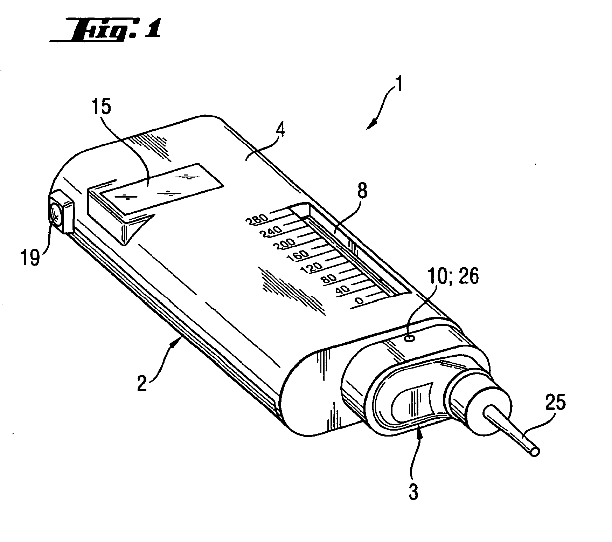

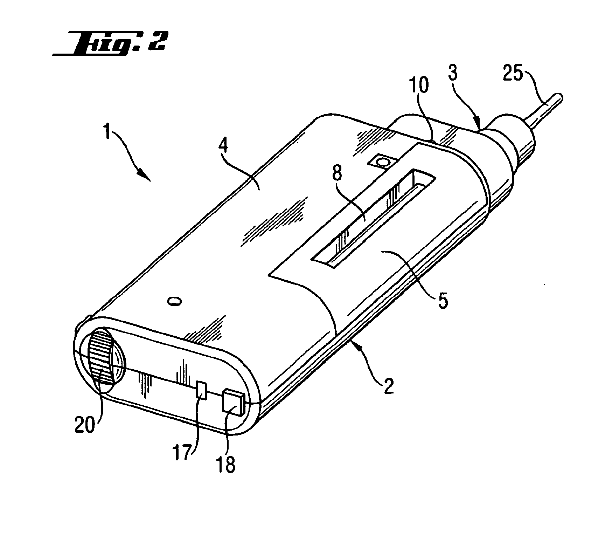

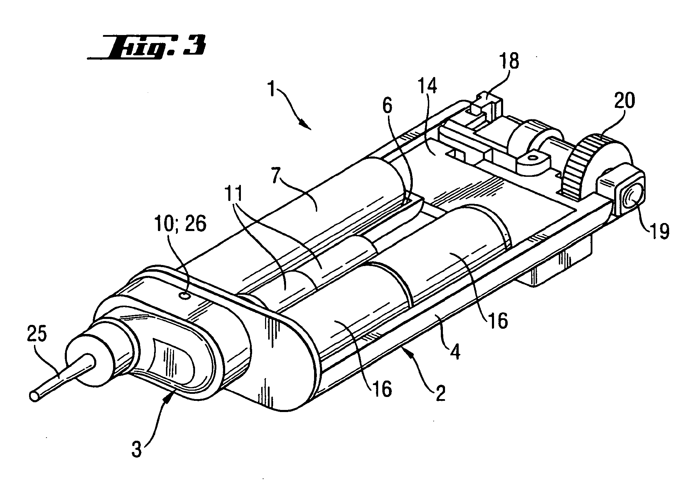

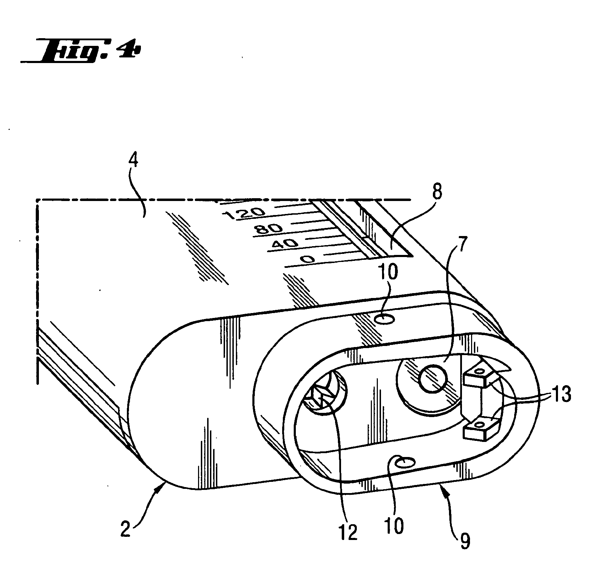

Der Insulinpen besteht aus einem Hauptgerät mit wechselbarem Pumpenkopf. Das Hauptgerät ist wieder verwendbar. Es besteht aus einem Gehäuse, in dem Pumpenantrieb, Sensorik, Elektronik und Energieversorgung untergebracht sind (Fig. 1; Fig. 2; Fig. 3)).. Weiter ist es mit Schnittstellen zu externen Geräten sowie mit einem Start- und Dosierknopf versehen. Der Pumpenkopf ist ein Wegwerfteil und wird nur über einen kurzen Zeitraum (1-3 Tage) eingesetzt. Er besitzt Schnittstellen zum Hauptgerät sowie zur Pennadel (Fig. 4).

### Wechselbarer Pumpenkopf

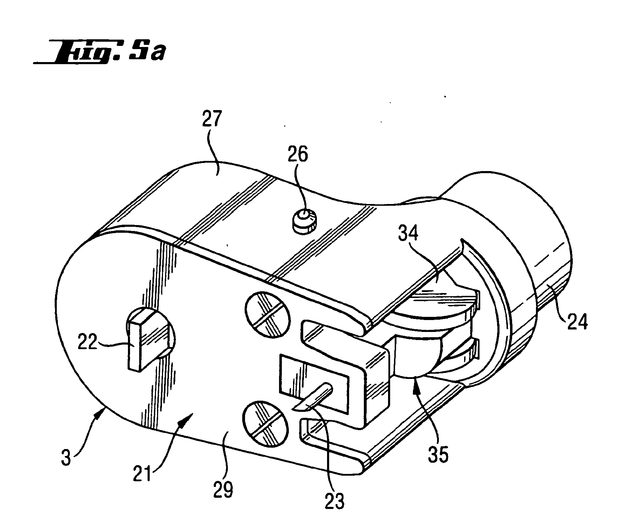

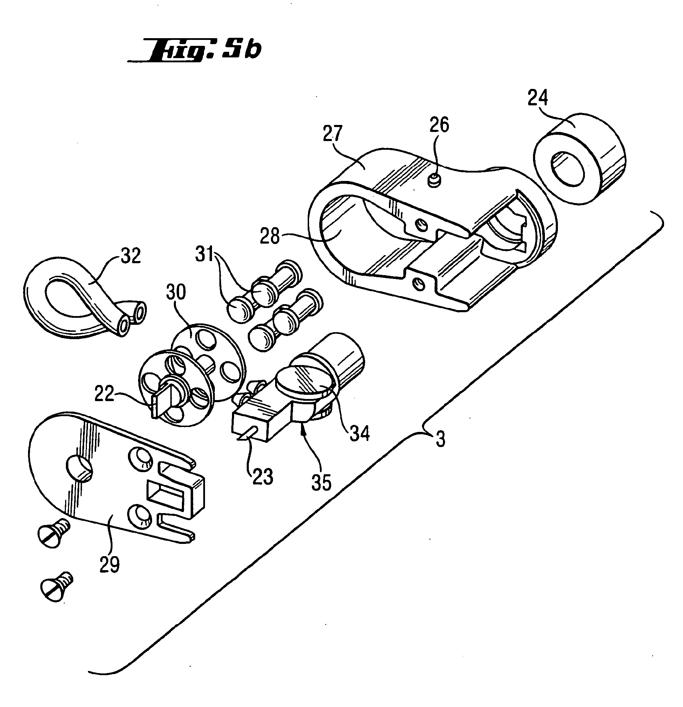

Der Pumpenkopf besteht aus einer Pumpenkammer (Schlauchpumpe) und einem Flusssensor (Flügelradzähler), die in einem Gehäuse untergebracht sind. Das Gehäuse besitzt Schnittstellen, die leicht trennbar bzw. wieder schließbar sind (Fig. 5).

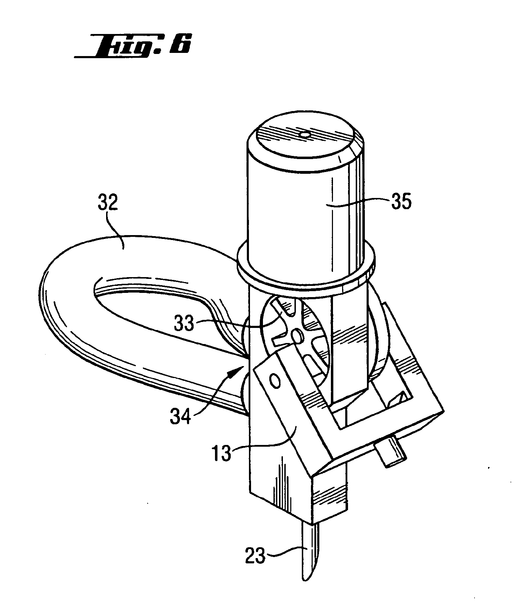

Der Flusssensor ist in dieser Ausführungsform in zwei Komponenten getrennt. Im Pumpenkopf befindet sich ein kostengünstig herzustellenden Flügelrad (Messobjekt). Dieses wird zusammen mit dem Pumpenkopf gewechselt. Die Rotation des Rades wird mit einer Gabellichtschranke erfasst, welche fest im Hauptgerät integriert ist (Fig. 6). Der Flusssensor kann auch einteilig vorliegen, wobei er entweder im Pumpenkopf integriert oder getrennt von diesem ist.

Liste der Figuren:
Figure 1: Ansicht Insulinpen von vorn (Maße: ca. 120 mm x 45 mm x 20 mm)
Figure 2: Ansicht Insulinpen von hinten
Figure 3: Einzelkomponenten des Insulinpens
Figure 4: Schnittstelle zum Pumpenkopf
Figure 5: Wechselbarer Pumpenkopf
Figure 6: Flügelrad mit Lichtschranke

Erklärung der Bezugsnummern
- 1: Insulinpen
- 2: Grundkörper (Unterseite)
- 3: Wechselbarer Pumpenkopf
- 4: Grundkörper (Oberseite)
- 5: Deckel zu Ampullenfach
- 6: Ampullenfach
- 7: Ampulle
- 8: Ampullensichtfenster
- 9: Anschlussstück des Grundkörpers zur Aufnahme des wechselbaren Pumpenkopfes
- 10: Halterung zwischen Grundkörper und wechselbarem Pumpenkopf
- 11: Motor
- 12: Motorkupplung
- 13: Lichtschranke
- 14: Elektronik mit LCD (rückseitig)
- 15: LCD Anzeige
- 16: Fotobatterie
- 17: Schnittstelle zu PC
- 18: Ein/Aus Schalter
- 19: Startknopf
- 20: Dosierknopf
- 21: Anlagefläche des wechselbaren Pumpenkopfes zum Grundkörper
- 22: Kupplung zur Pumpe
- 23: Schnittstelle zur Ampulle
- 24: Schnittstelle zur Nadel
- 25: Nadel
- 26: Halterung zwischen Grundkörper und wechselbarem Pumpenkopfes
- 27: Basisteil des wechselbaren Pumpenkopfes (Außenseite)
- 28: Basisteil des wechselbaren Pumpenkopfes (Innenteil)
- 29: Abdeckteil des wechselbaren Pumpenkopfes
- 30: Rotor
- 31: Rollen
- 32: Schlauch
- 33: Flügelrand
- 34: Flusssensor
- 35: Fluidteil

## Patentansprüche

1. Vorrichtung (1) zum Bewegen von Flüssigkeiten, die ein Arzneimittel enthalten, die technische Vorrichtung umfassend wenigstens
a) einen Motor (11);
b) einen Vorratsbehälter (6, 7);
c) einen Pumpenkopf (3), der vom Motor (11) aus a) angetrieben wird und mittels dessen die Flüssigkeit aus dem Vorratsbehälter (6, 7) abgeführt wird;
d) eine Steuerelektronik (14);
**gekennzeichnet dadurch, dass** der Pumpenkopf (3) aus c) mit lösbaren und funktionell wieder verbindbaren Schnittstellen (9, 10, 12, 22, 23, 26) zum Motor (11) aus a) und dem Vorratsbehälter (6, 7) aus b) ausgestattet ist.

2. Technische Vorrichtung (1) nach Anspruch 1, **gekennzeichnet dadurch, dass** der Pumpenkopf (3) weiterhin mit einer lösbaren und wieder verbindbaren Schnittstelle (13, 33, 34) zur Steuerelektronik (14) ausgestattet ist.

3. Technische Vorrichtung (1) nach Anspruch 1 oder 2, **gekennzeichnet dadurch, dass** der Pumpenkopf (3) gegen einen anderen Pumpenkopf (3) ohne Verwendung eines Werkzeugs auswechselbar ist.

4. Technische Vorrichtung (1) nach einem oder mehreren der Ansprüche 1 bis 3, **gekennzeichnet dadurch, dass** der Pumpenkopf (3) einen Flusssensor (34) und/oder Bauteile des Flusssensors (13, 33) beinhaltet.

5. Technische Vorrichtung (1) nach einem oder mehreren der Ansprüche 1 bis 4, **gekennzeichnet dadurch, dass** der Pumpenkopf (3) eine Nadel (25) trägt.

6. Technische Vorrichtung (1) nach einem oder mehreren der Ansprüche 1 bis 5, **gekennzeichnet dadurch, dass** der Motor (11) aus einem Mikromotor besteht.

7. Technische Vorrichtung (1) nach einem oder mehreren der Ansprüche 1 bis 6, **gekennzeichnet dadurch, dass** die technische Vorrichtung (1) eingerichtet ist, eine Ampulle (7) zur Aufnahme eines Medikaments als Vorratsbehälter (6, 7) auswechselbar aufzunehmen.

8. Technische Vorrichtung (1) nach einem oder mehreren der Ansprüche 1 bis 7, **gekennzeichnet dadurch, dass** der Vorratsbehälter (6, 7) Insulin enthält.

9. Technische Vorrichtung (1) nach einem oder mehreren der Ansprüche 1 bis 8, **gekennzeichnet dadurch, dass** der Vorratsbehälter (6, 7) separat vom Pumpenkopf (3), insbesondere in einer Einheit, umfassend den Motor (11) und die Steuerelektronik (14), bereitgestellt ist.

10. Herstellung einer Vorrichtung (1) gemäß einem oder mehrerer der Ansprüche 1 bis 9, wobei
a) ein Motor (11) bereitgestellt wird;
b) ein Vorratsbehälter (6, 7) bereitgestellt wird;
c) ein Pumpenkopf (3) bereitgestellt wird;
d) die Einzelbestandteile wie in a) bis c) beschrieben zu einer funktionellen Einheit (1) zusammengebaut werden.

11. Medizinisches Gerät (1) zur Injektion eines Arzneimittels in den menschlichen oder tierischen Körper umfassend unter anderem
a) ein Gehäuse (2, 4, 5);
b) einen Einstellmechanismus (20) zur Voreinstellung einer durch das medizinische Gerät (1) abzugebenden Menge des Arzneimittels;
c) eine Anzeige (15);
d) eine technische Einheit (19) in Gestalt eines Auslösemechanismus zum Ingangsetzen und Durchführen der Injektion;
**gekennzeichnet dadurch, dass** zusätzlich mindestens eine Vorrichtung (1) gemäß einem oder mehreren der Ansprüche 1 bis 9 enthalten ist.

12. Medizinisches Gerät (1) nach Anspruch 11, **gekennzeichnet dadurch, dass** die Anzeige (15) aus einer LCD Anzeige (15) besteht.

13. Medizinisches Gerät (1) nach Anspruch 11 oder 12, **gekennzeichnet dadurch, dass** mindestens ein Mittel (14) zur Speicherung und/oder Verarbeitung von Daten und/oder Signalen sowie mindestens eine Schnittstelle (17) zur Übertragung von Daten und/oder Signalen zu und/oder von einer externen technischen Einheit, welche zur Speicherung und/oder Verarbeitung von Daten und/oder Signalen ausgelegt ist, enthalten ist.

14. Medizinisches Gerät (1) nach Anspruch 13, **gekennzeichnet dadurch, dass** die externe technische Einheit aus einem PC besteht, auf welchem ein Programm zur Speicherung und/oder Verarbeitung von Daten und/oder Signalen installiert ist.

15. Medizinisches Gerät (1) gemäß einem oder mehreren der Ansprüche 11 bis 14, **gekennzeichnet dadurch, dass** der zur Injektion vorgesehene Stoff aus Insulin besteht.

16. Medizinisches Gerät (1) gemäß Anspruch 15, worin das Insulin ein langwirksames und/oder kurzwirksames Insulin ist.

17. Medizinisches Gerät (1) gemäß einem oder mehreren der Ansprüche 11 bis 14, **gekennzeichnet dadurch, dass** der zur Injektion vorgesehene Stoff aus GLP-1 besteht.

18. Medizinisches Gerät (1) gemäß einem oder mehreren der Ansprüche 11 bis 14, **gekennzeichnet dadurch, dass** der zur Injektion vorgesehene Stoff aus einem Heparin besteht.

19. Herstellung eines medizinischen Geräts (1) zur Injektion eines Arzneimittels in den menschlichen oder tierischen Körper gemäß einem oder mehreren der Ansprüche 11 bis 18, wobei
a) ein Gehäuse (2, 4, 5) bereitgestellt wird;
b) ein Einstellmechanismus (20) zur Voreinstellung einer durch das medizinische Gerät (1) abzugebenden Menge eines Arzneimittels bereitgestellt ist;
c) eine Anzeige (15) bereitgestellt wird;
d) ein Auslösemechanismus (19) bereitgestellt wird;
e) evtl. elektronische Bestandteile (14) bereitgestellt werden;
f) eine technische Vorrichtung (1) gemäß einem oder mehreren der Ansprüche 1 bis 9 bereitgestellt wird;
g) die Einzelbestandteile wie in a) bis f) beschrieben zu einer funktionellen Einheit (1) zusammengebaut werden.

## Claims

1. A device (1) for moving liquids which comprise a pharmaceutical, the technical device comprising at least
a) a motor (11);
b) a reservoir (6, 7);
c) a pump head (3) which is driven by the motor (11) from a) and by means of which the liquid is conveyed out of the reservoir (6, 7);
d) control electronics (14);
wherein the pump head (3) from c) is equipped with detachable and functionally reconnectable interfaces (9, 10, 12, 22, 23, 26) to the motor (11) from a) and to the reservoir (6, 7) from b).

2. The technical device (1) as claimed in claim 1, wherein the pump head (3) is furthermore equipped with a detachable and reconnectable interface (13, 33, 34) to the control electronics (14).

3. The technical device (1) as claimed in claim 1 or 2, wherein the pump head (3) can be exchanged for another pump head (3) without using a tool.

4. The technical device (1) as claimed in one or more of claims 1 to 3, wherein the pump head (3) comprises a flow sensor (34) and/or components of the flow sensor (13, 33).

5. The technical device (1) as claimed in one or more of claims 1 to 4, wherein the pump head (3) carries a needle (25).

6. The technical device (1) as claimed in one or more of claims 1 to 5, wherein the motor (11) consists of a micromotor.

7. The technical device (1) as claimed in one or more of claims 1 to 6, wherein the technical device (1) is configured to exchangeably receive a cartridge (7) for receiving a medicament, as the reservoir (6, 7).

8. The technical device (1) as claimed in one or more of claims 1 to 7, wherein the reservoir (6, 7) comprises insulin.

9. The technical device (1) as claimed in one or more of claims 1 to 8, wherein the reservoir (6, 7) is provided separately from the pump head (3), in particular in a unit comprising the motor (11) and the control electronics (14).

10. The production of a device (1) as claimed in one or more of claims 1 to 9, where
a) a motor (11) is provided;
b) a reservoir (6, 7) is provided;
c) a pump head (3) is provided;
d) the individual constituents as described in a) to c) are assembled to give a functional unit (1).

11. A medical apparatus (1) for injecting a pharmaceutical into the human or animal body, comprising inter alia
a) a housing (2, 4, 5);
b) an adjusting mechanism (20) for presetting an amount of the pharmaceutical to be delivered by the medical apparatus (1);
c) a display (15);
d) a technical unit (19) in the form of a release mechanism for starting up and carrying out the injection;
which additionally comprises at least one device (1) as claimed in one or more of claims 1 to 9.

12. The medical apparatus (1) as claimed in claim 11, wherein the display (15) consists of an LCD display (15).

13. The medical apparatus (1) as claimed in claim 11 or 12, which comprises at least one means (14) for storing and/or processing data and/or signals, and at least one interface (17) for transmitting data and/or signals to and/or from an external technical unit which is configured to store and/or process data and/or signals.

14. The medical apparatus (1) as claimed in claim 13, wherein the external technical unit consists of a PC on which a program for storing and/or processing data and/or signals is installed.

15. The medical apparatus (1) as claimed in one or more of claims 11 to 14, wherein the substance intended for injection consists of insulin.

16. The medical apparatus (1) as claimed in claim 15, in which the insulin is a long-acting and/or short-acting insulin.

17. The medical apparatus (1) as claimed in one or more of claims 11 to 14, wherein the substance intended for injection consists of GLP-1.

18. The medical apparatus (1) as claimed in one or more of claims 11 to 14, wherein the substance intended for injection consists of a heparin.

19. The production of a medical apparatus (1) for injecting a pharmaceutical into the human or animal body as claimed in one or more of claims 11 to 18, where
a) a housing (2, 4, 5) is provided;
b) an adjusting mechanism (20) is provided to preset an amount of a pharmaceutical which is to be delivered by the medical apparatus (1);
c) a display (15) is provided;
d) a release mechanism (19) is provided;
e) possibly electronic constituents (14) are provided;
f) a technical device (1) as claimed in one or more of claims 1 to 9 is provided;
g) the individual constituents as described in a) to f) are assembled to give a functional unit (1).

## Revendications

1. Dispositif (1) pour déplacer des liquides, qui contiennent un médicament, le dispositif technique comprenant au moins
a) un moteur (11),
b) un réservoir de stockage (6, 7),
c) une tête de pompe (3), qui est entraînée par le moteur (11) de a) et au moyen de laquelle le liquide est expulsé hors du réservoir de stockage (6, 7),
d) une électronique de commande (14),
**caractérisé en ce que** la tête de pompe (3) de c) est équipée d'interfaces (9, 10, 12, 22, 23, 26), pouvant être détachées et de nouveau fonctionnellement connectées, vers le moteur (11) de a) et vers le réservoir de stockage (6, 7) de b).

2. Dispositif technique (1) selon la revendication 1, **caractérisé en ce que** la tête de pompe (3) est en outre équipée d'une interface (13, 33, 34), pouvant être détachée et de nouveau fonctionnellement connectée, vers l'électronique de commande (14).

3. Dispositif technique (1) selon la revendication 1 ou 2, **caractérisé en ce que** la tête de pompe (3) peut être échangée contre une autre tête de pompe (3) sans utilisation d'un outil.

4. Dispositif technique (1) selon une ou plusieurs des revendications 1 à 3, **caractérisé en ce que** la tête de pompe (3) comporte un détecteur d'écoulement (34) et/ou des composants du détecteur d'écoulement (13, 33).

5. Dispositif technique (1) selon une ou plusieurs des revendications 1 à 4, **caractérisé en ce que** la tête de pompe (3) porte une aiguille (25).

6. Dispositif technique (1) selon une ou plusieurs des revendications 1 à 5, **caractérisé en ce que** le moteur (11) est constitué par un micro-moteur.

7. Dispositif technique (1) selon une ou plusieurs des revendications 1 à 6, **caractérisé en ce que** le dispositif technique (1) est conçu pour recevoir, de façon échangeable comme réservoir de stockage (6, 7), une ampoule (7) destinée à contenir un médicament.

8. Dispositif technique (1) selon une ou plusieurs des revendications 1 à 7, **caractérisé en ce que** le réservoir de stockage (6, 7) contient de l'insuline.

9. Dispositif technique (1) selon une ou plusieurs des revendications 1 à 8, **caractérisé en ce que** le réservoir de stockage (6, 7) est préparé séparément de la tête de pompe (3), en particulier en une unité comprenant le moteur (11) et l'électronique de commande (14).

10. Fabrication d'un dispositif (1) selon une ou plusieurs des revendications 1 à 9, dans laquelle
a) on prépare un moteur (11),
b) on prépare un réservoir de stockage (6, 7),
c) on prépare une tête de pompe (3),
d) on assemble les composants individuels décrits sous a) à c) en une unité fonctionnelle (1).

11. Appareil médical (1) destiné à l'injection d'un médicament dans le corps humain ou animal, comprenant notamment
a) un boîtier (2, 4, 5);
b) un mécanisme de réglage (20) pour le préréglage d'une quantité de médicament à administrer au moyen de l'appareil médical (1);
c) un écran d'affichage (15);
d) une unité technique (19) sous la forme d'un mécanisme de déclenchement pour le démarrage et l'exécution de l'injection;
**caractérisé en ce que** qu'il comprend en outre au moins un dispositif (1) selon une ou plusieurs des revendications 1 à 9.

12. Appareil médical (1) selon la revendication 11, **caractérisé en ce que** l'écran d'affichage (15) est constitué d'un écran LCD (15).

13. Appareil médical (1) selon la revendication 11 ou 12, **caractérisé en ce qu'**il comprend au moins un moyen (14) pour mémoriser et/ou traiter des données et/ou des signaux ainsi qu'au moins une interface (17) pour la transmission de données et/ou de signaux en direction et/ou en provenance d'une unité technique externe, qui est conçue en vue de mémoriser et/ou de traiter des données et/ou des signaux.

14. Appareil médical (1) selon la revendication 13, **caractérisé en ce que** l'unité technique externe se compose d'un PC, sur lequel est installé un programme pour mémoriser et/ou traiter des données et/ou des signaux.

15. Appareil médical (1) selon une ou plusieurs des revendications 11 à 14, **caractérisé en ce que** la substance prévue pour l'injection se compose d'insuline.

16. Appareil médical (1) selon la revendication 15, dans lequel l'insuline est une insuline lente et/ou une insuline rapide.

17. Appareil médical (1) selon une ou plusieurs des revendications 11 à 14, **caractérisé en ce que** la substance prévue pour l'injection se compose de GLP-1.

18. Appareil médical (1) selon une ou plusieurs des revendications 11 à 14, **caractérisé en ce que** la substance prévue pour l'injection se compose d'une héparine.

19. Fabrication d'un appareil médical (1) destiné à l'injection d'un médicament dans le corps humain ou animal selon une ou plusieurs des revendications 11 à 18, dans laquelle
a) on prépare un boîtier (2, 4, 5);
b) on prépare un mécanisme de réglage (20) pour le préréglage d'une quantité d'un médicament à administrer au moyen de l'appareil médical (1);
c) on prépare un écran d'affichage (15);
d) on prépare un mécanisme de déclenchement (19);
e) on prépare des composants électroniques (14);
f) on prépare un dispositif technique (1) selon une ou plusieurs des revendications 1 à 9;
g) on assemble les composants individuels décrits sous a) à f) en une unité fonctionnelle (1).
